(19) Europäisches Patentamt
European Patent Office
Office européen des brevets

(11) **EP 3 776 573 B1**

## (12) EUROPÄISCHE PATENTSCHRIFT

(45) Veröffentlichungstag und Bekanntmachung des Hinweises auf die Patenterteilung:
**27.11.2024   Patentblatt 2024/48**

(21) Anmeldenummer: **19718608.3**

(22) Anmeldetag: **09.04.2019**

(51) Internationale Patentklassifikation (IPC):
***G16H 20/40*** *(2018.01)*

(52) Gemeinsame Patentklassifikation (CPC):
**G16H 20/17; G16H 20/40; G16H 30/40; G16H 40/63**

(86) Internationale Anmeldenummer:
**PCT/EP2019/058919**

(87) Internationale Veröffentlichungsnummer:
**WO 2019/197387 (17.10.2019 Gazette 2019/42)**

(54) **VORRICHTUNG UND VERFAHREN ZUR BESTIMMUNG VON RESTBLUT IN EINEM DIALYSATOR SOWIE DIALYSESYSTEM**

DEVICE AND METHOD FOR DETERMINING RESIDUAL BLOOD IN A DIALYSER AND DIALYSIS SYSTEM

DISPOSITIF ET PROCÉDÉ DE DÉTERMINATION DE SANG RÉSIDUEL DANS UN DIALYSEUR AINSI QUE SYSTÈME DE DIALYSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.04.2018   DE 102018205387**

(43) Veröffentlichungstag der Anmeldung:
**17.02.2021   Patentblatt 2021/07**

(73) Patentinhaber: **Fresenius Medical Care Deutschland GmbH**
**61352 Bad Homburg (DE)**

(72) Erfinder:
• **ERLENKÖTTER, Ansgar**
**66606 St. Wendel (DE)**
• **UTZIG, Lukas**
**66606 St. Wendel (DE)**

(74) Vertreter: **Ricker, Mathias**
**Wallinger Ricker Schlotter Tostmann**
**Patent- und Rechtsanwälte Partnerschaft mbB**
**Zweibrückenstraße 5-7**
**80331 München (DE)**

(56) Entgegenhaltungen:
EP-A1- 2 322 907      EP-A2- 1 078 642
JP-A- 2004 113 894

**Beschreibung**

Die vorliegende Erfindung betrifft eine Vorrichtung und ein Verfahren zur Bestimmung von Restblut in einem Dialysator sowie ein Dialysesystem.

Zur Reinigung von Blut eines Patienten sind Dialysesysteme bekannt, bei welchen in einem sog. Dialysator ein Filter aus einer Vielzahl von Hohlfasern, deren Wand durch eine semipermeable Membran gebildet wird, angeordnet ist. Während in den Fasern das Blut geführt wird, wird der Außenraum von einem sog. Dialysat durchströmt, so dass kleine Moleküle wie etwa Wasser, Elektrolyte und harnpflichtige Substanzen aus dem Blut durch die Membran in das Dialysat treten und abtransportiert werden können.

Bei der Dialyse kann eine unerwünschte Aktivierung einer Koagulation des Blutes durch Verwendung eines Antikoagulans (z.B. Heparin) verhindert oder zumindest vermindert werden. Wenn die Dosis des Antikoagulans zu niedrig ist, kann es während der Behandlung zu einer Gerinnselbildung kommen. Diese Gerinnsel können einzelne oder aber viele der Hohlfasern blockieren. Wenn das Blut am Ende der Behandlung dem Patienten wieder zugeführt wird, kann es vorkommen, dass eine gewisse Menge an Blut, welches auch als "Restblut" bezeichnet wird, in den betreffenden Fasern verbleibt und nicht an den Patienten zurückgegeben wird.

Die optimale Dosis des Antikoagulans wird in der klinischen Praxis durch verschiedene Methoden identifiziert. Eine Möglichkeit ist die Bestimmung von Koagulationszeiten (z.B. ACT = aktivierte Gerinnungszeit, aPTT = aktivierte partielle Thromboplastinzeit) von Blutproben, die während der Behandlung gezogen werden (z.B. bei t = 60 min). Eine verlängerte Koagulationszeit zeigt die Wahl einer zu hohen Antikoagulationsdosis an, eine reduzierte Koagulationszeit dagegen eine zu niedrige Dosis. Ferner kann auch die Blutungszeit, die notwendig ist, damit der Patient nach dem Entfernen der Nadel aufhört zu bluten, dazu verwendet werden, die optimale Dosis Antikoagulans abzuschätzen.

Nicht zuletzt kann die sog. "Streifigkeit" der eingesetzten Filter durch Fachpersonal begutachtet werden. In Falle eines am Ende der Reinfusion im Wesentlichen weißen Filters sind nicht blockierte, freie Fasern weiß, die von Restblut blockierten dagegen rot. Die Begutachtung der Filter kann jedoch zu einer sehr subjektiven Einstufung führen, so dass unterschiedliche Personen zu unterschiedlichen Schlussfolgerungen und Ergebnissen kommen können.

Aus der EP 1 078 642 A2 ist ein Verfahren zur Bestimmung von geronnenem Blut in einem Dialysator bekannt, bei dem die Anwesenheit von geronnenem Blut in Bezug auf einen Druckabfall nach dem Dialysator festgestellt wird.

Die EP 2 322 907 A1 offenbart ein Verfahren zum Detektieren der stirnseitigen Randflächen von Hohlfasern. Die Hohlfasern bilden mit einer zwischen ihnen befindlichen Bindemasse ein Hohlfaserbündel und reflektieren Testlicht stärker diffus als die Bindemasse. Mittels einer Testkamera wird Testlicht nach diffuser Reflexion an den stirnseitigen Randflächen der Hohlfasern erfasst und so ein Diffuslichtbild erzeugt, in welchem die stirnseitigen Randflächen der Hohlfasern heller erscheinen als die Bindemasse und anhand dieser Aufhellung detektiert werden. Weiterhin offenbart diese Druckschrift ein Verfahren zum Detektieren der Innenräume von nicht verstopften Hohlfasern eines solchen Hohlfaserbündels. Prüflicht wird an der Bindemasse glänzend reflektiert und danach mit einer Prüfkamera erfasst. So wird ein Glanzlichtbild erzeugt, in welchem die Innenräume von nicht verstopften Fasern dunkler erscheinen als die Bindemasse und die Innenräume von verstopften Hohlfasern. Anhand dieser Abdunklung werden die Innenräume nicht verstopfter Hohlfasern detektiert.

Die JP 2004 113894 A1 offenbart ein Verfahren, mit dem genau beurteilt werden kann, ob ein Hohlfasermembranmodul bei der Herstellung des Membranmoduls akzeptabel ist oder nicht. Hierzu wird eine vergossene Endfläche eines zu prüfenden Hohlfasermembranmoduls abgebildet, und aus den erhaltenen Daten werden regionale Informationen über den Teil, an dem eine Hohlfasermembran vorhanden ist, und den Teil, an dem ein Harz vorhanden ist, aus dem Bild gewonnen. Aus zwei Arten von regionalen Informationen wird die Fläche der Hohlfasermembran mit den geschlossenen Endteilen ermittelt. Wenn die Fläche einen vorgegebenen Schwellenwert überschreitet, wird das geprüfte Modul als defekt eingestuft.

Es ist eine Aufgabe der Erfindung, eine Vorrichtung und ein Verfahren zur zuverlässigen Bestimmung von Restblut in einem Dialysator sowie ein entsprechendes Dialysesystem anzugeben.

Eine erfindungsgemäße Vorrichtung zur Bestimmung von Restblut, insbesondere Blutkoagel, in einem Dialysator weist auf: eine Aufnahmeeinrichtung, die dazu eingerichtet ist, einen Dialysator aufzunehmen, eine Bilderfassungseinrichtung, die dazu eingerichtet ist, mindestens ein Bild eines von der Aufnahmeeinrichtung aufgenommenen Dialysators, insbesondere eines im Dialysator befindlichen Filters, zu erfassen und entsprechende Bilddaten zu erzeugen, welche eine Vielzahl von Bildpunkten (Pixel) aufweisen, denen jeweils mindestens ein Intensitätswert, insbesondere ein Farbwert, zugeordnet ist, und eine Steuerungseinrichtung, die dazu eingerichtet ist, eine das Vorhandensein und/oder den Gehalt von Restblut im Dialysator charakterisierende Restblutinformation basierend auf einer statistischen Häufigkeit zumindest eines Teils der Intensitätswerte in dem mindestens einen Bild zu bestimmen.

Ein erfindungsgemäßes Dialysesystem weist die erfindungsgemäße Vorrichtung sowie eine Dialysevorrichtung auf, welche mittels Schläuchen mit einem Dialysator verbindbar ist und dazu eingerichtet ist, Blut und Dialysat zur Reinigung des Blutes durch den Dialysator zu leiten.

Ein erfindungsgemäßes Verfahren zur Bestimmung von Restblut, insbesondere Blutkoagel, in einem Dialysator weist folgende Schritte auf: Erfassen mindestens eines Bildes eines Dialysators, insbesondere eines im Dialysator befindlichen Filters, und Erzeugen entsprechender Bilddaten, welche eine Vielzahl von Bildpunkten (Pixel) aufweisen, denen jeweils mindestens ein Intensitätswert, insbesondere ein Farbwert, zugeordnet ist, und Bestimmen einer das Vorhandensein und/oder den Gehalt von Restblut im Dialysator charakterisierenden Restblutinformation basierend auf einer statistischen Häufigkeit zumindest eines Teils der Intensitätswerte in dem mindestens einen Bild.

Ein Aspekt der Erfindung basiert auf dem Ansatz, mittels einer Kamera ein oder mehrere Bilder des im Dialysator befindlichen Filters aufzunehmen und das Vorhandensein und/oder den Gehalt von etwaigem Restblut, insbesondere koaguliertem Blut (sog. Blutkoagel), im Filter zu detektieren bzw. zu bestimmen, indem zumindest ein Teil der Grauwerte und/oder Farbwerte des erfassten Bildes bzw. der erfassten Bilder hinsichtlich ihrer statistischen Häufigkeit im jeweiligen Bild analysiert werden und daraus eine entsprechende Restblutinformation abgeleitet wird, die angibt, ob bzw. wieviel Restblut im Filter vorhanden ist. Bei der analysierten statistischen Häufigkeit der Grau- bzw. Farbwerte im Bild kann es sich um absolute Häufigkeitswerte handeln, welche die Anzahl der im Bild jeweils vorkommenden Grau- bzw. Farbwerte einer bestimmten Höhe angeben. Alternativ oder zusätzlich kann es sich bei der analysierten statistischen Häufigkeit um relative Häufigkeitswerte handeln, welche ein Maß für den Anteil der im Bild jeweils vorkommenden Grau- bzw. Farbwerte einer bestimmten Höhe an der im Bild insgesamt vorkommenden und/oder möglichen Anzahl von Grau- bzw. Farbwerten darstellen.

Die automatisierte Analyse der mittels Kamera erfassten Bilder des Filters mittels statistischer Methoden ermöglicht eine objektive und damit besonders zuverlässige Bestimmung von etwaigem Restblut in einem Dialysator. Ein weiterer Vorteil der Erfindung liegt auch darin, dass anhand der auf diese Weise bestimmten Restblutinformation die Dosierungsmenge eines Antikoagulans besonders zuverlässig bestimmt bzw. optimiert werden kann.

Vorzugsweise ist die Bilderfassungseinrichtung dazu eingerichtet, elektromagnetische Strahlung bei einer oder mehreren unterschiedlichen Wellenlängen oder Wellenlängenbereichen zu erfassen und je Bildpunkt Intensitätswerte, insbesondere zwei oder mehrere Farbwerte, für jede der Wellenlängen bzw. jeden der Wellenlängenbereiche zu erzeugen. Die Steuerungseinrichtung ist hierbei ferner dazu eingerichtet ist, die Restblutinformation basierend auf einer statistischen Häufigkeit der Intensitätswerte für eine oder mehrere der Wellenlängen bzw. für einen oder mehrere der Wellenlängenbereiche in dem mindestens einen Bild zu bestimmen. Vorzugsweise weist die Bilderfassungseinrichtung für jede der unterschiedlichen Wellenlängen bzw. für jeden der unterschiedlichen Wellenlängenbereiche einen sog. Farbkanal, in welchem je Bildpunkt ein entsprechender Farbwert bestimmt wird. Vorzugsweise weist die Bilderfassungseinrichtung drei Farbkanäle für die Wellenlängen bzw. Wellenlängenbereiche Rot, Grün und Blau auf. Die dabei erhaltenen Bilder werden auch als RGB-Bilder bezeichnet. Alternativ kann die Bilderfassungseinrichtung aber auch jede andere Kombination von unterschiedlichen Farbkanälen aufweisen, wie z.B. "Cyan", "Magenta", "Yellow" und den Schwarzanteil "Key", wodurch Bilder im sog. CMYK-Farbraum erhalten werden. Alternativ oder zusätzlich ist bevorzugt, dass die Bilderfassungseinrichtung eine Auflösung der Intensitätswerte, insbesondere der verschiedenen Farbwerte, von mindestens 100, insbesondere mindestens 200, Intensitätswerten aufweist. Beispielsweise ist für jeden der Kanäle eine Auflösung von 8 Bit, was 256 verschiedenen Intensitätswerten entspricht, vorgesehen. Die mittels einer oder mehrerer der vorstehend aufgeführten Ausgestaltungen erhaltenen bzw. ausgewerteten Bilder erlauben eine besonders zuverlässige Bestimmung von Restblut im Dialysator.

Es ist ferner bevorzugt, dass die Bilderfassungseinrichtung dazu eingerichtet ist, je Bild mehr als 250.000, insbesondere mehr als 1.000.000 Bildpunkte (Pixel) zu erzeugen. Aufgrund der dabei erzielten hohen Ortsauflösung der aufgenommenen Bilder lässt sich insbesondere in den Hohlfasern eingelagertes Restblut besonders zuverlässig bestimmen. Grundsätzlich brauchen jedoch nicht alle von der Bilderfassungseinrichtung erzeugten Bildpunkte eines Bildes bei der Bildanalyse berücksichtigt zu werden. Vielmehr ist es möglich, nur einen Bildausschnitt entsprechend zu analysieren. Liefert die Bilderfassungseinrichtung etwa ein Bild mit rund 1.000.000 Bildpunkten, so kann beispielsweise lediglich ein Bildausschnitt von rund 500.000 Bildpunkten einer statistischen Bildanalyse unterzogen werden, wenn sich z.B. die relevante Bildinformation, etwa der Filter oder ein repräsentativer Ausschnitt des Filters, in diesem Bildausschnitt wiedergegeben ist. Das spart Rechenkapazitäten, ohne dass die Zuverlässigkeit bei der Bestimmung von Restblut beeinträchtigt wird.

Grundsätzlich kann es sich bei den von der Bilderfassungseinrichtung erzeugten Bildern um Einzelbilder, Bildsequenzen und/oder aber auch um bewegte Bilder, insbesondere Videosequenzen, handeln.

Es ist ferner bevorzugt, dass die Steuerungseinrichtung dazu eingerichtet ist, die Restblutinformation basierend auf einer Verteilung der Häufigkeit, insbesondere einem Histogramm, der Intensitätswerte in dem mindestens einen Bild zu bestimmen. Allgemein kann durch ein Histogramm die Verteilung der Helligkeitswerte eines Bildes visualisiert werden, wobei über einer Achse, die den Wertebereich der Grau- bzw. Farbwerte darstellt, als Balken die einzelnen Häufigkeiten des Vorkommens dieser Grau- bzw. Farbwerte aufgetragen sind. Je höher der Balken über einem Grau- bzw. Farbwert ist, desto häufiger kommt dieser Grau- bzw. Farbwert im Bild vor. Die bei der vorliegenden Bestimmung von Restblut herangezogene Verteilung der Häufigkeit bzw. das Histogramm der Intensitätswerte braucht jedoch nicht notwendigerweise visualisiert zu werden, sondern kann lediglich die entsprechenden Datenpaare (Grau- bzw. Farbwert und absolute bzw. relative Häufigkeit) zur Verteilung der, insbesondere absoluten oder relativen, statistischen Häufigkeit der Grauwerte bzw. Farbwerte in einem Bild enthalten. Im Falle eines Bildes, das mit einer Bilderfassungseinrichtung mit zwei oder mehreren Farbkanälen aufgenommen worden ist, werden bei der statistischen Analyse des Bildes vorzugsweise entsprechend zwei oder mehrere Histogramme über die einzelnen Farbkanäle berücksichtigt, z.B. drei Histogramme bei einem RGB-Bild und vier Histogramme bei einem CMYK-Bild.

Die Steuerungseinrichtung ist vorzugsweise dazu eingerichtet ist, die Restblutinformation basierend auf mindestens einem Parameter, welcher eine Eigenschaft der Verteilung der Häufigkeit der Intensitätswerte in dem mindestens einen Bild charakterisiert, zu bestimmen. Anhand des vorzugsweise automatisiert bestimmten Parameters der Häufigkeitsverteilung der Grau- bzw. Farbwerte eines Bildes kann etwaiges Restblut im Dialysator bzw. Filter mit noch größerer Zuverlässigkeit bestimmt werden. Dies gilt insbesondere auch für einen oder mehrere der nachfolgend beispielhaft aufgeführten Parameter.

Vorzugsweise beinhaltet bzw. charakterisiert der mindestens eine Parameter den Erwartungswert der Intensitätswerte bzw. den Flächenschwerpunkt der Verteilung der Häufigkeit der Intensitätswerte in dem mindestens einen Bild, insbesondere den Erwartungswert bzw. Flächenschwerpunkt der Farbwerte im blauen und/oder grünen Wellenlängenbereich bzw. der Kanäle Blau und/oder Grün in dem mindestens einen Bild. Der Erwartungswert gibt vorzugsweise den Intensitätswert an, bei welchem der Flächenschwerpunkt einer von der Verteilung der Häufigkeit der Intensitätswerte über der Abszisse (x-Achse) gebildeten Fläche liegt. Vorzugsweise ist die Steuerungseinrichtung dazu eingerichtet, die den Gehalt, insbesondere eine Menge, von Restblut im Dialysator charakterisierende Restblutinformation basierend auf dem ermittelten Erwartungswert bzw. Flächenschwerpunkt zu ermitteln.

Alternativ oder zusätzlich beinhaltet bzw. charakterisiert der mindestens eine Parameter einen Intensitätswert, bei welchem die Verteilung der Häufigkeit der Intensitätswerte ein Maximum aufweist. Alternativ oder zusätzlich beinhaltet bzw. charakterisiert der mindestens eine Parameter einen Intensitätswert, bei welchem die Verteilung der Häufigkeit der Intensitätswerte in zwei gleich große Flächen geteilt wird.

Vorzugsweise charakterisiert der mindestens eine Parameter die Breite der Verteilung der Häufigkeit der Intensitätswerte in dem mindestens einen Bild. Insbesondere charakterisiert der mindestens eine Parameter mindestens eines der folgenden: die Standardabweichung der Intensitätswerte vom Erwartungswert und/oder die Varianz der Intensitätswerte und/oder eine Spannweite zwischen zwei Intensitätswerten (Cut-on, Cut-off), bei welchen der Verlauf der Verteilung der Häufigkeit einen vorgegebenen Schwellenwert, insbesondere Null, überschreitet und/oder erreicht. Anhand des die Breite der Verteilung charakterisierenden Parameters kann die Steuereinrichtung zuverlässig automatisch ermitteln, ob ein Filter einheitlich gefärbt ist bzw. es weiße Bereiche ohne Bluteinlagerung gibt und/oder der Filter eine Streifigkeit zeigt, um daraus die Restblutinformation abzuleiten. Alternativ kann die Restblutinformation aber auch direkt aus der Breite der Verteilung abgeleitet werden.

Vorzugsweise charakterisiert der mindestens eine Parameter die Schiefe der Verteilung der Häufigkeit der Intensitätswerte in dem mindestens einen Bild, insbesondere der Farbwerte im roten Wellenlängenbereich bzw. des Kanals Rot. Insbesondere ist die Steuerungseinrichtung dazu eingerichtet, die den Gehalt, insbesondere eine Menge, von Restblut im Dialysator charakterisierende Restblutinformation basierend auf der, insbesondere für den roten Wellenlängenbereich, Schiefe der Verteilung zu ermitteln. Insbesondere wird hierbei geprüft, ob die Verteilung der Häufigkeit rechtsschief oder linksschief ist, wobei im Falle einer rechtsschiefen Verteilung auf einen weißen Filter ohne nennenswerte Bluteinlagerungen und im Falle einer linksschiefen Verteilung auf einen Filter mit Bluteinlagerungen geschlossen wird.

Vorzugsweise charakterisiert der mindestens eine Parameter die Kurtosis der Verteilung der Häufigkeit der Intensitätswerte. Bei der Kurtosis handelt es sich vorzugsweise um ein Maß für die Spitzheit oder Spitzgipfeligkeit einer Verteilung, insbesondere bezogen auf eine Normalverteilung. Sind z.B. in der im Verlauf der Verteilung der Häufigkeit mehrere Peaks oder Schultern enthalten, so nimmt der Wert der Kurtosis ab. Vorzugsweise ist die Steuerungseinrichtung dazu eingerichtet, die den Gehalt, insbesondere eine Menge, von Restblut im Dialysator charakterisierende Restblutinformation basierend auf der Kurtosis zu ermitteln, wobei insbesondere bei einem geringeren Wert der Kurtosis auf einen weißen Filter ohne nennenswerte Bluteinlagerung und bei einem höheren Wert der Kurtosis auf eine entsprechende Menge an Restblut im Filter geschlossen werden kann.

Vorzugsweise erfolgt das Bestimmen der Restblutinformation ferner unter Berücksichtigung von Kalibrierdaten, die in einem Kalibrierverfahren ermittelt werden, das folgende Schritte aufweist: Erfassen von Bildern von zwei oder mehreren Dialysatoren, insbesondere von in den Dialysatoren befindlichen Filtern, in welchen unterschiedliche Mengen an Restblut enthalten sind, und Erzeugen entsprechender Bilddaten, welche eine Vielzahl von Bildpunkten aufweisen, denen jeweils mindestens ein Intensitätswert, insbesondere ein Farbwert, zugeordnet ist, und Erzeugen von Kalibrierdaten basierend auf einer Information bezüglich der in den Dialysatoren jeweils enthaltenen unterschiedlichen Mengen an Restblut und einer Analyse der statistischen Häufigkeit zumindest eines Teils der Intensitätswerte in den jeweils erfassten Bildern.

Bei dem vorzugsweise vor dem eigentlichen Bestimmen der Restblutinformation durchgeführten Kalibrierverfahren werden vorzugsweise mehrere Bilder, z.B. 5, 10 oder 20 Bilder, an unterschiedlichen Stellen und/oder bei unterschiedlichen Lagen, z.B. unterschiedlichen Rotationsstellungen, des jeweiligen Dialysators bzw. Filters, in welchem jeweils eine bekannte Menge an Restblut enthalten ist, aufgenommen. Beispielsweise werden die Bilder von drei unterschiedlich gefüllten Dialysatoren aufgenommen, welche z.B. nur mit NaCl-Lösung (ohne Blut), ganz mit Blut bzw. nur zur Hälfte mit Blut gefüllt sind. Die von den unterschiedlich gefüllten Dialysatoren aufgenommenen Bilddaten werden jeweils einer Auswertung unterzogen, bei welcher die statistische Häufigkeit der in den Bildern jeweils vorkommenden Intensitätswerte, insbesondere verschiedener Farbwerte wie etwa in den Farbkanälen Rot, Grün und Blau, ermittelt und vorzugsweise mittels zumindest eines der vorstehend beschriebenen Parameter charakterisiert wird, wie z.B. durch den Erwartungswert, die Breite, Schiefe und/oder Kurtosis der jeweiligen Verteilung der Häufigkeit der Farbwerte.

Beispielsweise enthalten die Kalibrierdaten für jeden der unterschiedlich gefüllten Dialysatoren zumindest einen Parameter bzw. einen entsprechenden Intensitätswert je Farbkanal. Bei z.B. drei unterschiedlichen Dialysatoren (nur NaCl, halb mit Blut gefüllt, ganz mit Blut gefüllt) und z.B. drei Farbkanälen (Rot, Grün, Blau) werden je Dialysator drei Erwartungswerte der Farbwerte bei Rot, Grün und Blau erhalten.

Bei der späteren Bestimmung von Restblut in einem Dialysator mit unbekannter Restblutmenge können dann diese Kalibrierdaten herangezogen werden, indem der Erwartungswert der Farbwerte in den Farbkanälen Rot, Grün und Blau des von diesem Dialysator erfassten Bildes bestimmt und mit den für unterschiedlich gefüllte Dialysatoren erhaltenen Farbwert-Tripletts der Kalibrierdaten verglichen wird. Alternativ ist es auch möglich, die Erwartungswerte der Farbwerte von nur zwei Farbkanälen oder nur einem Farbkanal zu ermitteln bzw. zu vergleichen. So wurde überraschenderweise gefunden, dass die Bestimmung des Erwartungswertes für den Farbkanal Grün und dessen Vergleich mit den bei der Kalibrierung für unterschiedlich gefüllte Dialysatoren erhaltenen Erwartungswerten für den Farbkanal Grün eine besonders einfache und dennoch zuverlässige Bestimmung von Restblut im jeweiligen Dialysator ermöglicht.

Weitere Vorteile, Merkmale und Anwendungsmöglichkeiten der vorliegenden Erfindung ergeben sich aus der nachfolgenden Beschreibung in Zusammenhang mit den Figuren. Es zeigen:

Fig. 1    ein Beispiel einer Vorrichtung zur Bestimmung von Restblut in einem Dialysator;

Fig. 2    ein erstes Beispiel einer Verteilung der Häufigkeit von Intensitätswerten in einem Bild eines mit NaCl gefüllten Dialysators; und

Fig. 3    ein zweites Beispiel einer Verteilung der Häufigkeit von Intensitätswerten in einem Bild eines zur Hälfte mit Blut gefüllten Dialysators;

Fig. 4    ein drittes Beispiel einer Verteilung der Häufigkeit von Intensitätswerten in einem Bild eines ganz mit Blut gefüllten Dialysators;

Fig. 5    eine graphische Darstellung eines Beispiels von Kalibrierdaten; und

Fig. 6    eine graphische Darstellung zur Veranschaulichung verschiedener Parameter zur Charakterisierung einer Verteilung der Häufigkeit von Intensitätswerten.

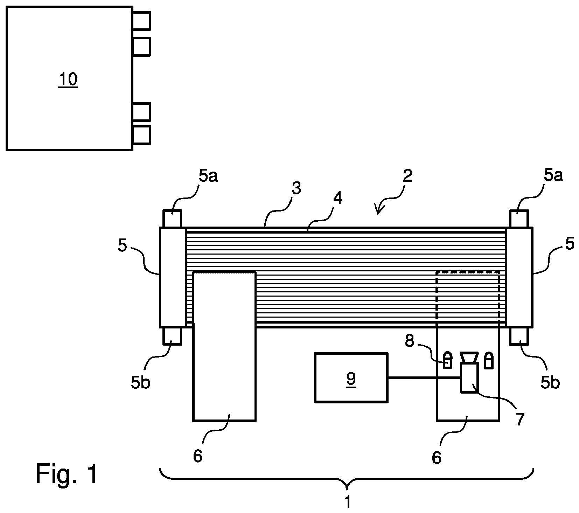

Figur 1 zeigt ein Beispiel einer Vorrichtung 1 zur Bestimmung von Restblut in einem Dialysator 2 in einer schematischen Seitenansicht. Der Dialysator 2 weist einen im Wesentlichen zylinderförmigen Mantel 3 aus einem vorzugsweise transparenten Material, wie etwa Kunststoff oder Glas, auf. Die beiden Enden des Mantels 3 sind mit Verschlusskappen 5 versehen, an welchen sich jeweils Anschlüsse 5a und 5b befinden, an welchen Blut und Dialysat in das Innere bzw. aus dem Inneren des Dialysators 2 geführt werden können, wenn diese über (nicht dargestellte) Schläuche mit entsprechenden Anschlüssen einer Dialysevorrichtung 10 verbunden werden.

Im Inneren des Dialysators 2 bzw. des Mantels 3 befindet sich ein Filter 4, welcher im dargestellten Beispiel eine Vielzahl von nur schematisch angedeuteten Hohlfasern aus einem semipermeablen Material aufweist. Während des Dialysevorgangs wird das zu reinigende Blut im Inneren der Hohlfasern geführt, während diese von außen mit Dialysat gespült werden.

Im dargestellten Beispiel befindet sich der Dialysator 2 - z.B. nach Beendigung eines Dialysevorgangs bzw. nach Trennung von der Dialysevorrichtung 10 - in einer Aufnahmeeinrichtung, welche zwei Lagerungselemente 6 mit halbzylindrischen Ausnehmungen aufweist, in denen der Dialysator 2, insbesondere drehbar, gelagert ist.

Im Innern eines der Lagerungselemente 6, welches im Querschnitt dargestellt ist, ist eine Bilderfassungseinrichtung 7, beispielsweise eine Standbild- und/oder Videokamera, vorgesehen, mittels welcher ein oder mehrere Bilder eines im Bereich des Lagerungselements 6 befindlichen Ausschnitts des Dialysators 2 bzw. Filters 4 aufgenommen werden können. Vorzugsweise dient ein Kamerachip als Bilderfassungseinrichtung 7.

Vorzugsweise ist ferner eine Beleuchtungseinrichtung 8 vorgesehen, z.B. eine oder mehrere Leuchtdioden, mittels welcher der betreffende Ausschnitt des Dialysators 2 bzw. Filters 4 zumindest während der Bilderfassung beleuchtet wird.

**EP 3 776 573 B1**

In Falle einer drehbaren Lagerung des Dialysators 2 in den Lagerungselementen 6 kann vorgesehen sein, dass mehrere Bilder des Dialysators 2 nacheinander bei unterschiedlichen Drehstellungen aufgenommen werden. Insbesondere kann vorgesehen sein, dass durch Drehung des Dialysators 2 ein Panoramabild aufgenommen wird.

Die Bilderfassungseinrichtung 7 ist vorzugsweise zur Erfassung von Farbbildern ausgelegt und weist z.B. drei Farbkanäle in den Wellenlängen bzw. Wellenlängenbereichen Rot (R), Grün (G) und Blau (B) auf.

Jedes von der Bilderfassungseinrichtung 7 aufgenommene Bild des Dialysators 2 bzw. Filters 4 weist eine Vielzahl von Bildpunkten, sog. Pixel, auf, welchen jeweils mindestens ein Intensitätswert, insbesondere drei unterschiedliche Farbwerte für die Farbkanäle R, G und B, zugeordnet ist bzw. sind.

Die von der Bilderfassungseinrichtung 7 erzeugten Bilddaten werden einer Steuerungseinrichtung 9 zugeführt und dort analysiert, um Rückschlüsse auf etwaiges Restblut im Filter 4 zu gewinnen.

Dabei wird zumindest ein Teil der Farbwerte hinsichtlich ihrer Häufigkeit, insbesondere hinsichtlich ihrer Häufigkeitsverteilung, im jeweiligen Bild analysiert und daraus eine entsprechende Restblutinformation abgeleitet, die angibt, ob bzw. wieviel Restblut im Filter 4 vorhanden ist.

Die Restblutinformation kann lediglich qualitativer Natur sein, z.B. "Restblut vorhanden" bzw. "kein Restblut vorhanden", oder aber auch quantitative Informationen zum Restblutgehalt enthalten, z.B. in Form von groben Angaben zum Restblutgehalt (z.B. "hoch", "mittel", "gering") und/oder aber auch in Form von Zahlenwerten (z.B. Gewichtsprozent, Volumenprozent, absolute Konzentrationswerte, Angaben zur sog. Streifigkeit des Filters).

Die Restblutinformation wird vorzugsweise unter Berücksichtigung zumindest eines Parameters bestimmt, welcher zumindest eine Eigenschaft der Häufigkeitsverteilung wenigstens eines der Farbwerte charakterisiert. Vorzugsweise werden dabei zusätzlich vorab ermittelte Kalibrierdaten, in welchen eine oder mehrere Eigenschaften der Häufigkeitsverteilung mit dem Blutgehalt korreliert sind, berücksichtigt. Dies wird im Folgenden näher erläutert.

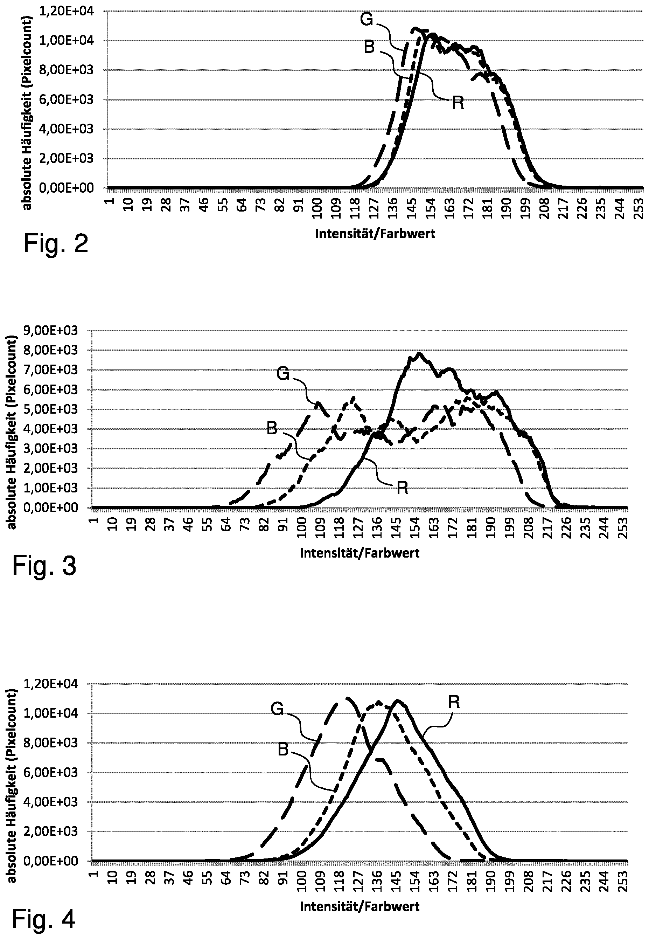

Figur 2 zeigt ein erstes Beispiel einer Verteilung der Häufigkeit von Intensitätswerten in einem RGB-Bild eines mit NaCl gefüllten Dialysators. Bei der gezeigten graphischen Darstellung handelt es sich um ein sog. Histogramm, bei welchem die absolute Häufigkeit in Form der Anzahl von Bildpunkten (sog. Pixelcount) im jeweils aufgenommenen Bild über den in diesem Bild vorkommenden Intensitäts- bzw. Farbwerten in den einzelnen Farbkanälen Rot (R), Grün (G) und Blau (B) aufgetragen ist.

Figur 3 zeigt ein zweites Beispiel einer Verteilung der Häufigkeit von Intensitätswerten in einem RGB-Bild eines zur Hälfte mit Blut gefüllten Dialysators. Im Unterschied zu den in Figur 2 gezeigten Verteilungen der Farbwerte in den unterschiedlichen Kanälen sind die Verteilungen in der Figur 3 breiter. Die Verteilungen in den unterschiedlichen Kanälen R, G und B unterscheiden sich auch untereinander stärker als in Figur 2.

Figur 4 zeigt ein drittes Beispiel einer Verteilung der Häufigkeit von Intensitätswerten in einem Bild eines ganz mit Blut gefüllten Dialysators. Im Unterschied zu den in den Figuren 2 und 3 gezeigten Verteilungen der Farbwerte in den unterschiedlichen Kanälen sind die Verteilungen in der Figur 4 symmetrischer und deutlich spitzer. Ferner ist eine deutliche Verschiebung des Flächenschwerpunkts, sog. Centroid, der Verteilungen in den unterschiedlichen Kanälen R, G und B zu erkennen.

Im Rahmen einer Kalibrierung der Vorrichtung 1 bzw. des entsprechenden Verfahrens kann z.B. der Flächenschwerpunkt der Häufigkeitsverteilungen ermittelt und mit dem jeweiligen Blutgehalt korreliert werden. Die Lage des Flächenschwerpunkts $x_s$ einer Verteilung f(x) entlang der x-Achse ist wie folgt definiert:

$$x_s = \frac{\int_a^b x \cdot f(x)dx}{\int_a^b f(x)dx} = \frac{I_1}{I_2}$$

Um eine Berechnung mit diskreten Intensitätswerten (x-Achse) und Häufigkeitswerten (y-Achse) durchzuführen, wird die Gleichung folgendermaßen angepasst:

$$x_s = \frac{\sum_{i=0}^{255} \int_{x_i}^{x_{i+1}} x \cdot f(x)dx}{\sum_{i=0}^{255} \int_{x_i}^{x_{i+1}} f(x)dx} = \frac{I_1}{I_2}$$

Es gilt dann:
*mit*

$$f(x)\{x \in \mathbb{R} \mid x_i \leq x \leq x_{i+1}\} = const = Fw(i)$$

6

$$I_1 = \sum_{i=0}^{255} \left[ \frac{1}{2} \cdot Fw(i) \cdot x \right]_{x_i}^{x_{i+1}} = \sum_{i=0}^{255} \frac{1}{2} Fw(i) \cdot \left( x_{i+1}^2 - x_i^2 \right)$$

mit

$$x_{i+1} = x_i + 1$$

$$I_1 = \sum_{i=0}^{255} \frac{1}{2} Fw(i) \cdot \left( x_{i+1}^2 - x_i^2 \right) = \sum_{i=0}^{255} \frac{1}{2} Fw(i) \cdot \left( (x_i + 1)^2 - x_i^2 \right)$$

$$I_1 = \sum_{i=0}^{255} \frac{1}{2} Fw(i) \cdot \left( x_i^2 + 2x_i + 1 - x_i^2 \right)$$

$$I_1 = \sum_{i=0}^{255} Fw(i) \cdot \left( x_i + \frac{1}{2} \right)$$

$$I_2 = \sum_{i=0}^{255} [Fw(i) \cdot x]_{x_i}^{x_{i+1}} = \sum_{i=0}^{255} Fw(i) \cdot (x_i + 1 - x_i)$$

$$I_2 = \sum_{i=0}^{255} Fw(i)$$

$$x_s = \frac{\sum_{i=0}^{255} Fw(i) \cdot \left( x_i + \frac{1}{2} \right)}{\sum_{i=0}^{255} Fw(i)}$$

Diese Gleichung entspricht einer Bestimmung des Schwerpunktes aus den Histogrammdaten (absolut). Es ist auch möglich, die Bestimmung des Schwerpunkts $x_s$ aus Histogrammdaten (relativ) durchzuführen:

$$x_s = \sum_{i=0}^{255} Fw(i) \cdot h_n(i)$$

$h_n$: relative Häufigkeit

Auch eine Bestimmung des Schwerpunkts aus Pixelrohdaten ist möglich:

$$x_s = \bar{x} = \frac{1}{i} \sum_{i=0}^{255} p\,(i)$$

p(i): Pixelwert $\{ p \in \mathbb{R} \mid 0 \leq x \leq 255 \}$

Auf diese Weise kann aus den in den Figuren 2 bis 4 beispielhaft gezeigten Verteilungen der unterschiedlichen Farbwerte eine Korrelation zwischen dem Intensitätswert bzw. Farbwert im jeweiligen Flächenschwerpunkt einerseits und der unterschiedlichen Blutfüllung des Dialysators andererseits hergestellt werden.

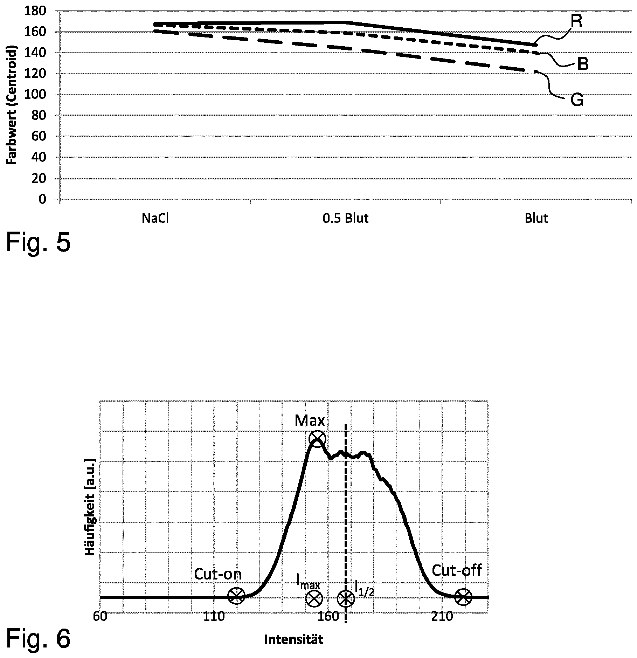

Die auf diese Weise für einem bestimmten Dialysator- bzw. Filtertyp beispielhaft erhaltenen Kalibrierdaten sind in Figur 5 graphisch veranschaulicht. Wie aus der Darstellung ersichtlich ist, ändern sich die für den Farbkanal Grün (G) erhaltenen Intensitätswerte der Flächenschwerpunkte besonders deutlich in Abhängigkeit vom jeweiligen Blutgehalt. Entsprechendes gilt, wenn auch in etwas weniger ausgeprägter Form, auch für den Farbkanal Blau (B).

Bei der Bestimmung von Restblut in einem solchen Dialysator wird daher vorzugsweise die Häufigkeitsverteilung bzw. deren Flächenschwerpunkt für den Farbkanal Grün und/oder Blau und /oder Rot ermittelt. Durch Vergleich des dabei erhaltenen Intensitätswertes des Flächenschwerpunkts mit den Kalibrierdaten für den Farbkanal Grün bzw. Blau bzw. Rot kann dann auf den Restblutgehalt im Dialysator geschlossen werden. Die Verwendung des grünen Farbkanals ist bevorzugt.

Vorzugsweise werden die bei der Kalibrierung jeweils erhaltenen Wertepaare aus Intensitätswert und Blutgehalt, wie in Figur 5 gezeigt, linear interpoliert und/oder extrapoliert, so dass der Restblutgehalt auch für Blutmengen bestimmt werden kann, welche zwischen, unter und/oder über den bei der Kalibrierung berücksichtigten Blutmengen liegen.

Alternativ oder zusätzlich ist es auch möglich, den jeweiligen Flächenschwerpunkt der in den Figuren 2 bis 4 gezeigten Häufigkeitsverteilungen entlang der y-Achse (Häufigkeit) in entsprechender Weise zu bestimmen und bei der Bestimmung von Restblut zu berücksichtigen. In diesem Fall wird beim Kalibriervorgang entsprechend eine Korrelation zwischen der, insbesondere absoluten, Häufigkeit der Intensitätswerte und dem Blutgehalt hergestellt.

Während der Flächenschwerpunkt der Verteilungen entlang der x-Achse ein Maß für die Verschiebung der Häufigkeitsverteilungen der Intensitätswerte abhängig vom Blutgehalt darstellt, beschreibt der Flächenschwerpunkt entlang der y-Achse deren Gewichtung.

Alternativ oder zusätzlich zum Flächenschwerpunkt bzw. Erwartungswert können aber auch andere Eigenschaften der Häufigkeitsverteilungen bei der Bestimmung von Restblut bzw. der Kalibrierung berücksichtigt werden, wie z.B. die Breite, Schiefe und/oder Kurtosis der jeweiligen Verteilung, der Intensitätswert bzw. Farbwert, bei welchem die Häufigkeitsverteilung ein Maximum aufweist und/oder die Intensitätswerte, sog. Cut-on- bzw. Cut-off-Werte, bei welchen die jeweilige Verteilung die Nulllinie verlässt bzw. wieder erreicht.

Figur 6 zeigt eine graphische Darstellung der Häufigkeitsverteilung von Intensitätswerten eines Bildes zur Veranschaulichung verschiedener Parameter zur Charakterisierung der Eigenschaften der Verteilung.

Die eingezeichnete gestrichelte Gerade beim Intensitätswert $I_{1/2}$ teilt die von der Häufigkeitsverteilung über der x-Achse gebildete Fläche in zwei gleich große Flächen. Der Intensitätswert $I_{1/2}$ kann, alternativ oder zusätzlich zum Flächenschwerpunkt bzw. Erwartungswert, ebenfalls zur Bestimmung von Restblut herangezogen werden.

Die beiden Punkte "Cut-on" und "Cut-off" kennzeichnen die Intensitäts- und/oder Häufigkeitswerte im Verlauf der Häufigkeitsverteilung, an welchen die Intensität den Wert Null überschreitet bzw. wieder erreicht. Die entsprechenden Intensitätswerte können, alternativ oder zusätzlich zu den vorstehend beschriebenen Parametern, ebenfalls zur Bestimmung von Restblut herangezogen werden.

Dies gilt entsprechend auch für den Punkt "Max", welcher den am häufigsten vorkommenden Intensitätswert $I_{max}$ bzw. die Häufigkeit dieses Intensitätswertes $I_{max}$ kennzeichnet.

**Patentansprüche**

1. Vorrichtung (1) zur Bestimmung von Restblut in einem Dialysator (2) mit

   - einer Aufnahmeeinrichtung (6), die dazu eingerichtet ist, einen Dialysator (2) aufzunehmen,
   - einer Bilderfassungseinrichtung (7), die dazu eingerichtet ist, mindestens ein Bild eines von der Aufnahmeeinrichtung (6) aufgenommenen Dialysators (2), insbesondere eines im Dialysator (2) befindlichen Filters (4), zu erfassen und entsprechende Bilddaten zu erzeugen, welche eine Vielzahl von Bildpunkten aufweisen, denen jeweils mindestens ein Intensitätswert, insbesondere ein Farbwert, zugeordnet ist, und
   - einer Steuerungseinrichtung (9), die dazu eingerichtet ist, eine das Vorhandensein und/oder den Gehalt von Restblut im Dialysator (2) charakterisierende Restblutinformation basierend auf einer statistischen Häufigkeit zumindest eines Teils der Intensitätswerte in dem mindestens einen Bild zu bestimmen.

2. Vorrichtung (1) nach Anspruch 1, wobei

   - die Bilderfassungseinrichtung (7) dazu eingerichtet ist, elektromagnetische Strahlung bei zwei oder mehreren unterschiedlichen Wellenlängen zu erfassen und je Bildpunkt Intensitätswerte, insbesondere zwei oder mehrere Farbwerte, für jede der Wellenlängen zu erzeugen, und

- die Steuerungseinrichtung (9) dazu eingerichtet ist, die Restblutinformation basierend auf einer statistischen Häufigkeit der Intensitätswerte für eine oder mehrere der Wellenlängen in dem mindestens einen Bild zu bestimmen .

3. Vorrichtung (1) nach Anspruch 1 oder 2, wobei die Steuerungseinrichtung (9) dazu eingerichtet ist, die Restblutinformation basierend auf einer Verteilung der Häufigkeit, insbesondere einem Histogramm, der Intensitätswerte in dem mindestens einen Bild zu bestimmen.

4. Vorrichtung (1) nach Anspruch 3, wobei die Steuerungseinrichtung (9) dazu eingerichtet ist, die Restblutinformation basierend auf mindestens einem Parameter, welcher eine Eigenschaft der Verteilung der Häufigkeit der Intensitätswerte in dem mindestens einen Bild charakterisiert, zu bestimmen.

5. Vorrichtung (1) nach Anspruch 4, wobei der mindestens eine Parameter durch den Erwartungswert der Intensitätswerte und/oder den Flächenschwerpunkt der Verteilung der Häufigkeit der Intensitätswerte gegeben ist.

6. Vorrichtung (1) nach Anspruch 4 oder 5, wobei der mindestens eine Parameter durch einen Intensitätswert ($I_{max}$) gegeben ist, bei welchem die Verteilung der Häufigkeit der Intensitätswerte ein Maximum aufweist.

7. Vorrichtung (1) nach einem der Ansprüche 4 bis 6, wobei der mindestens eine Parameter durch einen Intensitätswert ($I_{1/2}$) gegeben ist, bei welchem die Verteilung der Häufigkeit der Intensitätswerte in zwei gleich große Flächen geteilt wird.

8. Vorrichtung (1) nach einem der Ansprüche 4 bis 7, wobei der mindestens eine Parameter die Breite der Verteilung der Häufigkeit der Intensitätswerte charakterisiert.

9. Vorrichtung (1) nach Anspruch 8, wobei der mindestens eine Parameter mindestens eines der folgenden charakterisiert:

   - die Standardabweichung der Intensitätswerte vom Erwartungswert,
   - die Varianz der Intensitätswerte,
   - eine Spannweite zwischen zwei Intensitätswerten (Cut-on, Cut-off), bei welchen der Verlauf der Verteilung der Häufigkeit einen vorgegebenen Schwellenwert, insbesondere Null, überschreitet und/oder erreicht.

10. Vorrichtung (1) nach einem der Ansprüche 4 bis 9, wobei der mindestens eine Parameter die Schiefe der Verteilung der Häufigkeit der Intensitätswerte charakterisiert.

11. Vorrichtung (1) nach einem der Ansprüche 4 bis 10, wobei der mindestens eine Parameter die Kurtosis der Verteilung der Häufigkeit der Intensitätswerte charakterisiert.

12. Dialysesystem mit

   - einer Vorrichtung (1) nach einem der vorangehenden Ansprüche und
   - einer Dialysevorrichtung (10), die mittels Schläuchen mit einem Dialysator (2) verbindbar ist und dazu eingerichtet ist, Blut und Dialysat zur Reinigung des Blutes durch den Dialysator (2) zu leiten.

13. Verfahren zur Bestimmung von Restblut in einem Dialysator (2) mit folgenden Schritten:

   - Erfassen mindestens eines Bildes eines Dialysators (2), insbesondere eines im Dialysator (2) befindlichen Filters (4), und Erzeugen entsprechender Bilddaten, welche eine Vielzahl von Bildpunkten aufweisen, denen jeweils mindestens ein Intensitätswert, insbesondere ein Farbwert, zugeordnet ist, und
   - Bestimmen einer das Vorhandensein und/oder den Gehalt von Restblut im Dialysator (2) charakterisierenden Restblutinformation basierend auf einer statistischen Häufigkeit zumindest eines Teils der Intensitätswerte in dem mindestens einen Bild.

14. Verfahren nach Anspruch 13, wobei das Bestimmen der Restblutinformation ferner unter Berücksichtigung von Kalibrierdaten erfolgt, welche mittels eines Kalibrierverfahrens erhalten werden, welches folgende Schritte aufweist:

   - Erfassen von Bildern von zwei oder mehreren Dialysatoren (2), insbesondere von in den Dialysatoren befindlichen Filtern (4), in welchen unterschiedliche Mengen an Restblut enthalten sind, und Erzeugen entsprechender Bilddaten, welche eine Vielzahl von Bildpunkten aufweisen, denen jeweils mindestens ein Intensitätswert, insbesondere mindestens ein Farbwert, zugeordnet ist, und

- Erzeugen von Kalibrierdaten basierend auf einer Information bezüglich der in den Dialysatoren jeweils enthaltenen unterschiedlichen Mengen an Restblut und einer Analyse der statistischen Häufigkeit zumindest eines Teils der Intensitätswerte in den jeweils erfassten Bildern.

**Claims**

1. An apparatus (1) for determining residual blood in a dialyzer (2) comprising

   - a holding device (6) configured to accommodate a dialyzer (2),
   - an image capturing device (7) configured to capture at least one image of a dialyzer (2) accommodated by the holding device (6), in particular a filter (4) located in the dialyzer (2), and generate corresponding image data having a plurality of pixels, each of which assigned at least one respective intensity value, in particular a color value, and
   - a control device (9) configured to determine residual blood information characterizing the presence and/or content of residual blood in the dialyzer (2) on the basis of a statistical frequency of at least a portion of the intensity values in the at least one image.

2. The apparatus (1) according to claim 1, wherein

   - the image capturing device (7) is configured to capture electromagnetic radiation at two or more different wavelengths and generate intensity values, in particular two or more color values, for each of the wavelengths per each pixel, and
   - the control device (9) is configured to determine the residual blood information on the basis of a statistical frequency of the intensity values for one or more of the wavelengths in the at least one image.

3. The apparatus (1) according to claim 1 or 2, wherein the control device (9) is configured to determine the residual blood information on the basis of a distribution of the frequency, in particular a histogram, of the intensity values in the at least one image.

4. The apparatus (1) according to claim 3, wherein the control device (9) is configured to determine the residual blood information on the basis of at least one parameter characterizing a characteristic of the frequency distribution of the intensity values in the at least one image.

5. The apparatus (1) according to claim 4, wherein the at least one parameter is given by the expected value of the intensity values and/or the centroid of the intensity value frequency distribution.

6. The apparatus (1) according to claim 4 or 5, wherein the at least one parameter is given by an intensity value ($I_{max}$) at which the frequency distribution of the intensity values exhibits a maximum.

7. The apparatus (1) according to one of claims 4 to 6, wherein the at least one parameter is given by an intensity value ($I_{1/2}$) at which the frequency distribution of the intensity values is divided into two equal areas.

8. The apparatus (1) according to one of claims 4 to 7, wherein the at least one parameter characterizes the width of the intensity value frequency distribution.

9. The apparatus (1) according to claim 8, wherein the at least one parameter characterizes at least one of the following:

   - the standard deviation of the intensity values from the expected value,
   - the variance of the intensity values,
   - a range between two intensity values (cut-on, cut-off) at which the progression of the frequency distribution exceeds and/or reaches a preset threshold, in particular zero.

10. The apparatus (1) according to one of claims 4 to 9, wherein the at least one parameter characterizes the slope to the intensity value frequency distribution.

**11.** The apparatus (1) according to one of claims 4 to 10, wherein the at least one parameter characterizes the kurtosis to the intensity value frequency distribution.

**12.** A dialysis system comprising

- an apparatus (1) according to one of the preceding claims and
- a dialysis apparatus (10) able to be connected to a dialyzer (2) by tubes and configured to conduct blood and dialysate through the dialyzer (2) to cleanse the blood.

**13.** A method for determining residual blood in a dialyzer (2) comprising the following steps:

- capturing at least one image of a dialyzer (2), in particular a filter (4) located in the dialyzer (2), and generating corresponding image data having a plurality of pixels, each of which assigned at least one respective intensity value, in particular a color value, and
- determining residual blood information characterizing the presence and/or content of residual blood in the dialyzer (2) on the basis of a statistical frequency of at least a portion of the intensity values in the at least one image.

**14.** The method according to claim 13, wherein the residual blood information is further determined in consideration of calibration data obtained by means of a calibration process which comprises the following steps:

- capturing images of two or more dialyzers (2), in particular of filters (4) located within the dialyzers containing different volumes of residual blood, and generating corresponding image data having a plurality of pixels, each of which associated with at least one intensity value, in particular at least one color value, and
- generating calibration data based on information on the respective differing amounts of residual blood contained in the dialyzers and an analysis of the statistical frequency of at least part of the intensity values in the respectively captured images.

**Revendications**

**1.** Dispositif (1) pour la détermination de sang résiduel dans un dialyseur (2) comprenant

- un système de réception (6), qui est conçu pour recevoir un dialyseur (2),
- un système d'acquisition d'image (7), qui est conçu pour acquérir au moins une image d'un dialyseur (2) reçu par le système de réception (6), en particulier d'un filtre (4) se trouvant dans le dialyseur (2), et produire des données d'image correspondantes, lesquelles présentent une pluralité de points d'image, auxquels est associée respectivement au moins une valeur d'intensité, en particulier une valeur chromatique, et
- un système de commande (9), qui est conçu pour déterminer une information de sang résiduel caractérisant la présence et/ou la teneur de sang résiduel dans le dialyseur (2) sur la base d'une fréquence statistique d'au moins une partie des valeurs d'intensité dans la au moins une image.

**2.** Dispositif (1) selon la revendication 1, dans lequel

- le système d'acquisition d'image (7) est conçu pour acquérir un rayonnement électromagnétique pour deux longueurs d'onde différentes ou plus et pour produire par point d'image des valeurs d'intensité, en particulier deux valeurs chromatiques ou plus, pour chacune des longueurs d'onde, et
- le système de commande (9) est conçu pour déterminer l'information de sang résiduel sur la base d'une fréquence statistique des valeurs d'intensité pour une ou plusieurs des longueurs d'onde dans la au moins une image.

**3.** Dispositif (1) selon la revendication 1 ou 2, dans lequel le système de commande (9) est conçu pour déterminer l'information de sang résiduel sur la base d'une distribution de la fréquence, en particulier d'un histogramme, des valeurs d'intensité dans la au moins une image.

**4.** Dispositif (1) selon la revendication 3, dans lequel le système de commande (9) est conçu pour déterminer l'information de sang résiduel sur la base d'au moins un paramètre, lequel caractérise une propriété de la distribution de la fréquence des valeurs d'intensité dans la au moins une image.

**5.** Dispositif (1) selon la revendication 4, dans lequel le au moins un paramètre est donné par la valeur escomptée des valeurs d'intensité et/ou le centroïde de la distribution de la fréquence des valeurs d'intensité.

**6.** Dispositif (1) selon la revendication 4 ou 5, dans lequel le au moins un paramètre est donné par une valeur d'intensité ($I_{max}$), pour laquelle la distribution de la fréquence des valeurs d'intensité présente un maximum.

**7.** Dispositif (1) selon l'une quelconque des revendications 4 à 6, dans lequel le au moins un paramètre est donné par une valeur d'intensité ($I_{1/2}$) < pour laquelle la distribution de la fréquence des valeurs d'intensité est divisée en deux surfaces de même taille.

**8.** Dispositif (1) selon l'une quelconque des revendications 4 à 7, dans lequel le au moins un paramètre caractérise la largeur de la distribution de la fréquence des valeurs d'intensité.

**9.** Dispositif (1) selon la revendication 8, dans lequel le au moins un paramètre caractérise au moins l'un de ce qui suit :

- l'écart-type des valeurs d'intensité par rapport à la valeur escomptée,
- la variance des valeurs d'intensité,
- une étendue entre deux valeurs d'intensité (Cut-on, Cut-off), pour lesquelles la variation de la distribution de la fréquence dépasse et/ou atteint une valeur de seuil prédéfinie, en particulier zéro.

**10.** Dispositif (1) selon l'une quelconque des revendications 4 à 9, dans lequel le au moins un paramètre caractérise la dissymétrie de la distribution de la fréquence des valeurs d'intensité.

**11.** Dispositif (1) selon l'une quelconque des revendications 4 à 10, dans lequel le au moins un paramètre caractérise l'aplatissement de la distribution de la fréquence des valeurs d'intensité.

**12.** Système de dialyse comprenant

- un dispositif (1) selon l'une quelconque des revendications précédentes et
- un dispositif de dialyse (10), qui peut être relié à un dialyseur (2) au moyen de tuyaux et est conçu pour guider le sang et le dialysat à travers le dialyseur (2) pour le nettoyage du sang.

**13.** Procédé pour la détermination de sang résiduel dans un dialyseur (2) comprenant les étapes suivantes :

- l'acquisition d'au moins une image d'un dialyseur (2), en particulier d'un filtre (4) se trouvant dans le dialyseur (2), et la génération de données d'image correspondantes, lesquelles présentent une pluralité de points d'image, auxquels est associée respectivement au moins une valeur d'intensité, en particulier une valeur chromatique, et
- la détermination d'une information de sang résiduel caractérisant la présence et/ou la teneur de sang résiduel dans le dialyseur (2) sur la base d'une fréquence statistique d'au moins une partie des valeurs d'intensité dans la au moins une image.

**14.** Procédé selon la revendication 13, dans lequel la détermination de l'information de sang résiduel s'effectue en outre avec prise en compte de données d'étalonnage, lesquelles sont obtenues au moyen d'un procédé d'étalonnage, lequel présente les étapes suivantes :

- l'acquisition d'images de deux dialyseurs (2) ou plus, en particulier de filtres (4) se trouvant dans les dialyseurs, dans lesquels sont contenues différentes quantités de sang résiduel, et la génération de données d'image correspondantes, lesquelles présentent une pluralité de points d'image, auxquels est associée respectivement au moins une valeur d'intensité, en particulier au moins une valeur chromatique, et
- la génération de données d'étalonnage sur la base d'une information concernant les différentes quantités de sang résiduel respectivement contenues dans les dialyseurs et une analyse de la fréquence statistique d'au moins une partie des valeurs d'intensité dans les images respectivement acquises.

Fig. 1

Fig. 2

Fig. 3

Fig. 4

Fig. 5

Fig. 6

**IN DER BESCHREIBUNG AUFGEFÜHRTE DOKUMENTE**

**In der Beschreibung aufgeführte Patentdokumente**

- EP 1078642 A2
- EP 2322907 A1
- JP 2004113894 A